# EUROPEAN PATENT APPLICATION

(11) **EP 1 176 142 A1**
(43) Date of publication of application: **30.01.2002**
(21) Application number: 01306346.6
(22) Date of filing: 24.07.2001
(51) Int. Cl.: C07D 231/14, C07D 231/38, C07D 487/04

(54) **Process for the preparation of pyrazoles**

(30) Priority: 28.07.2000 GB 0018662; 14.03.2001 GB 0106276
(71) Applicant: PFIZER INC., New York, N.Y. 10017 (US); Pfizer Limited, Sandwich, Kent CT13 9NJ (GB)
(72) Inventor: Harris, Laurence James, Sandwich, Kent CT13 9NJ (GB); Levett, Philip Charles, Sandwich, Kent CT13 9NJ (GB)
(74) Representative: Simpson, Alison Elizabeth Fraser

(57) **Abstract**

There is provided a process for the production of pyrazole compounds of general formula (II): 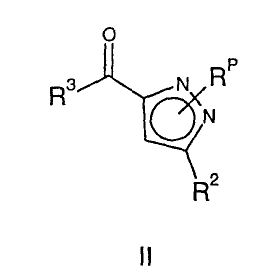 wherein R^{P} represents H or R¹; which process comprises the reaction of a compound of general formula (III), 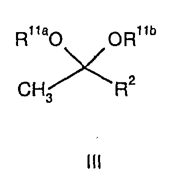 wherein R^{11a} and R^{11b} independently represent C₁-C₆ alkyl, R² is as defined herein before, with an acylating agent in the presence of a base, followed by *in situ* reaction with a hydrazine compound, wherein said process is optionally carried out in the presence of an activating agent.

## Description

This invention relates to a novel process for the preparation of pyrazoles. In particular the present invention relates to a novel process for the preparation of pyrazole intermediates useful in the synthesis of 4-alkylpiperazinylsulfonylphenyl- and 4-alkylpiperazinylsulfonyl pyridinyldihydropyrazolo[4,3-d]pyrimidin-7-one derivatives which are potent and selective cGMP PDE₅ inhibitors.

A general synthetic route for the preparation of 4-alkylpiperazinylsulfonylphenyl-dihydropyrazolo[4,3-d]pyrimidin-7-one derivatives (as described in EP 812 845, EP 994 115 and WO98/49166, and for analogues thereof as described in WO 99/54333). This synthesis involves a coupling reaction between an intermediate pyrazole compound (I) (as illustrated in scheme 1) and a phenyl or pyridinyl derivative followed by cyclisation of the resulting coupled intermediate to provide pyrimidin-7-ones.

We have now found that pyrazole compounds of general formula (II) which are used to prepare the intermediate pyrazole compounds (I) may be made *via* a novel "one-pot" process, as described hereinafter and illustrated in Scheme 1, which process has advantages over the multi-step processes for the preparation of compounds of general formula (II) as described in EP 812 845, EP 994 115, WO 98/49166 and WO 99/54333.

A further synthetic route for the preparation of certain pyrazole compounds of general formula (II) is described in Martins, M. A. P.; Freitag, R.; Flores, A. F. C.; Zanatta, N. *Synthesis,* 1995, 1491 and Martins, M. A. P.; Flores, A. F. C.; Zanatta, N.; Bastos, G. P.; Bonacorso, H. G.; Siqueira, G. M. *Tetrahedron Lett*. 1999, *40,* 4309. In this processes the pyrazole compounds are prepared in two steps.

According to a first aspect of the invention, there is provided a novel "one-pot" process for the production of pyrazole compounds of general formula (II): 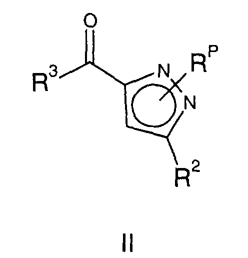 wherein R^{P} represents H or R¹;
R¹ represents: H, C₁-C₆ alkyl; C₁-C₆ alkoxy; C₃-C₆ cycloalkyl; C₁-C₆ alkyl(C₁-C₆ alkoxy), Het, C₁-C₆ alkylHet, aryl or C₁-C₆ alkylaryl, which latter eight groups are all optionally substituted (and/or, in the case of C₁-C₆ alkyl, optionally terminated) by one or more substituents selected from halo, cyano, nitro, C₁-C₆ alkyl, C(O)NR⁴R⁵, C(O)R⁶, C(O)OR⁷, OR⁸, NR^{9a}R^{9b} and SO₂NR^{10a}R^{10b};
R² represents C₁-C₆ alkyl; C₁-C₆ alkoxy; C₃-C₆ cycloalkyl; C₁-C₆ alkyl(C₁-C₆ alkoxy), Het, C₁-C₆ alkylHet, aryl or C₁-C₆ alkylaryl, which latter eight groups are all optionally substituted (and/or, in the case of C₁-C₆ alkyl, optionally terminated) by one or more substituents selected from halo, cyano, nitro, C₁-C₆ alkyl, C(O)NR⁴R⁵, C(O)R⁶, C(O)OR⁷, OR⁸, NR^{9a}R^{9b} and SO₂NR^{10a}R^{10b};
R³ represents: OH, C₁-C₆ alkoxy or NR⁴R⁵;
R⁴, R⁵, R⁶, R⁷, R⁸, R^{10a} and R^{10b} independently represent H or C₁-C₆ alkyl;
R^{9a} and R^{9b} either independently represent, H or C₁-C₆ alkyl or, together with the nitrogen atom to which they are attached, represent azetidinyl, pyrollidinyl or piperidinyl,
which process comprises the reaction of a compound of general formula (III), 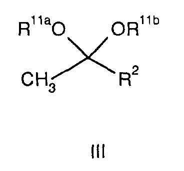 wherein R^{11a} and R^{11b} independently represent C₁-C₆ alkyl, R² is as defined herein before,
with an acylating agent of the formula (IV) in the presence of a base, 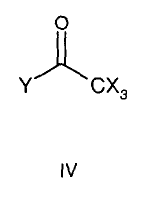 wherein X represents halogen independently selected from Cl, F or Br, and Y represents halogen or OR¹² wherein R¹² is: C₁-C₆ alkyl; C(O)CX₃; Het; or C₁-C₆ alkyl(Het) wherein Het is pyridine or imidazole,
followed by *in situ* reaction with a hydrazine compound of general formula (V) 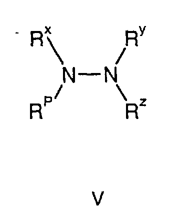 R^{P} represents H or R¹ wherein R¹ is as defined hereinbefore, and R^{x}, R^{y} and R^{z} are independently selected from H or electron donating groups (EDG) or electron withdrawing groups (EWG) wherein said electron withdrawing or donating groups are labile under the conditions of the reaction,
wherein said process is optionally carried out in the presence of an activating agent,
which process is referred to hereinafter as "the process of the invention". Advantageously, in the process according to the present invention the direct addition of the hydrazine compound (V) to the reaction vessel containing the reaction mixture of (III), (IV) and base results in production of compounds (II) with desirable purity and yield.

The compounds of general formula (II) can be represented by the formulae (IIA) and (IIB) as detailed hereinafter. The novel process according to the present invention includes the preparation of compounds of the formulae (IIA) and (IIB) as illustrated in Schemes 2 and 3.

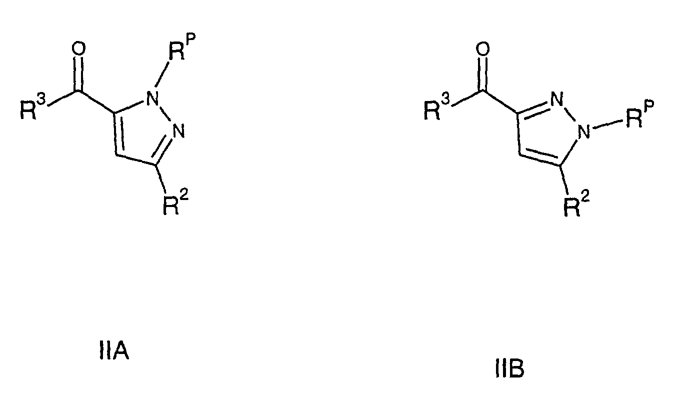

The term "aryl", when used herein, includes six- to ten-membered carbocyclic aromatic groups, such as phenyl and naphthyl and the like.

Het groups may be fully saturated, partly unsaturated, wholly aromatic, partly aromatic and/or bicyclic in character. Het groups that may be mentioned include groups such as optionally substituted azetidinyl, pyrrolidinyl, imidazolyl, indolyl, oxadiazolyl, thiadiazolyl, triazolyl, tetrazolyl, oxatriazolyl, thiatriazolyl, pyridazinyl, morpholinyl, pyrimidinyl, pyrazinyl, pyridyl, quinolinyl, isoquinolinyl, piperidinyl, pyrazolyl, imidazopyridinyl, piperazinyl, thienyl and furanyl.

The point of attachment of any Het group may be *via* any atom in the ring system including (where appropriate) a heteroatom. Het groups may also be present in the *N*- or *S*-oxidised form.

The term "C₁-C₆ alkyl" (which includes the C₁-C₆ alkyl part of alkylHet and alkylaryl groups), when used herein, includes (e.g. methyl, ethyl, propyl, butyl, pentyl and hexyl groups). Unless otherwise specified, C₁-C₆ alkyl groups may, when there is a sufficient number of carbon atoms, be linear or branched or be saturated or unsaturated.

As defined herein, the term "halo" includes fluoro, chloro, bromo and iodo.

Suitable electron donating groups, EDGs, for use herein include:
trialkylsilyl.

Suitable electron withdrawing groups, EWGs, for use herein include:
*tert*-butyloxycarbonyl and trifluoroacetamide.

Suitable bases for use herein preferably include: tertiary amines, such as triethylamine and di-*iso*-propylethylamine, 1,5-diazabicyclo[4.3.0]non-5-ene, 1,8-diazabicyclo[5.4.0]undec-7-ene, 1,4-diazabicyclo[2.2.2]octane, imidazole; substituted pyridines, such as 4-(dimethylamino)pyridine; benzofused pyridines, such as quinoline and isoquinoline; hindered metal alkoxides; hindered metal aryloxides; metal carbonates and bicarbonates.

Compounds of formulae (II) may contain one or more asymmetric carbon atoms and may therefore exhibit optical and/or diastereoisomerism. The process of the invention thus also relates to the formation of stereoisomers of compounds of formulae II and mixtures thereof. Stereoisomers may be separated using conventional techniques, e.g. chromatography or fractional crystallisation. The various stereoisomers may be isolated by separation of a racemic or other mixture of the compounds using conventional, e.g. fractional crystallisation or HPLC, techniques. Alternatively the desired optical isomers may be made by reaction of the appropriate optically active starting materials under conditions which will not cause racemisation or epimerisation, or by derivatisation, for example with a homochiral acid followed by separation of the diastereomeric esters by conventional means (e.g. HPLC, crystallisation, chromatography over silica or, for example, *via* classical resolution with a homochiral acid salt). The formation of all stereoisomers is included within the scope of the invention.

In a preferred aspect of the present invention there is provided a process for the production of pyrazole compounds of general formula (II): 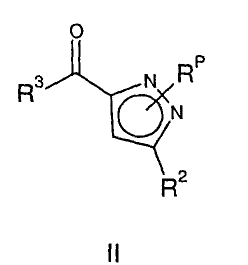 wherein R^{P} represents: H or R¹; R¹ represents C₁-C₄ alkyl, which alkyl group is optionally substituted as hereinbefore described; R² represents C₁-C₄ alkyl; R³ represents C₁-C₃ alkoxy, which process comprises the reaction of a compound (III), 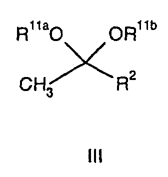 wherein R^{11a} and R^{11b} independently represent: C₁-C₄ alkyl and R² represents C₁-C₄ alkyl,
with an acylating agent of the formula (IV) in the presence of a base, preferably pyridine: 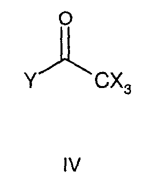 wherein X represents Cl or F and Y represents Cl, F or C(O)CX₃, optionally in the presence of an activating agent, followed by reaction with a hydrazine compound of general formula (V) in the same vessel: 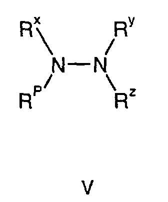 wherein R^{P} represents H or R¹ wherein R¹ is as defined hereinbefore, and R^{x}, R^{y} and R^{z} are independently selected from H or electron donating groups or electron withdrawing groups wherein said electron withdrawing or donating groups are labile under the conditions of the reaction.

In a further preferred aspect of the present invention there is provided a process for the production of pyrazole compounds of general formula (IIA): 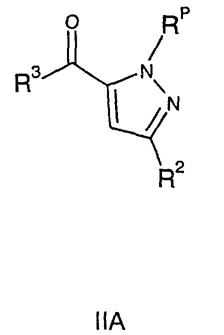 wherein R^{P} represents: H or R¹; R¹ represents C₁-C₄ alkyl, which alkyl group is optionally substituted as hereinbefore described; R² represents C₁-C₄ alkyl; R³ represents C₁-C₃ alkoxy, which process comprises the reaction of a compound (III): 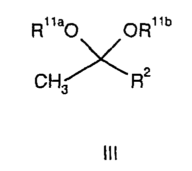 wherein R^{11a} and R^{11b} independently represent: C₁-C₄ alkyl and R² represents C₁-C₄ alkyl,
with an acylating agent in the presence of a base, preferably pyridine, of the formula (IV): 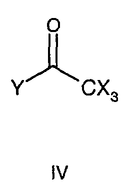 wherein X represents Cl or F and Y represents Cl, F or C(O)CX₃, optionally in the presence of an activating agent, followed by reaction with a hydrazine compound of general formula (V): 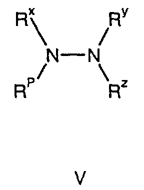 wherein R^{P} represents H or R¹ wherein R¹ is as defined hereinbefore, and
wherein R^{x} = EWG, R^{y} and R^{z} = H; or
R^{x} and R^{y} = H and R^{z} = EDG; or
R^{x} = R^{y} = R^{z} = H when R¹ = EWG; or
R^{x} = EWG, R^{y} = H and R^{z} = EDG
wherein EWG is a tri(C₁-C₂)alkylsilyl group and wherein EDG is selected from *tert*-butyloxycarbonyl and trifluoroacetamide.

In yet another preferred aspect of the present invention there is provided a process for the production of pyrazole compounds of general formula (IIB): 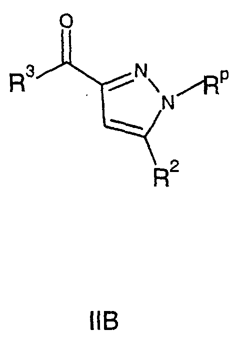 wherein R^{P} represents H or R¹ wherein R¹ represents C₁-C₄ alkyl or C₁-C₄ alkyl(C₁-C₂ alkoxy);
R² represents C₁-C₄ alkyl; and
R³ represents C₁-C₃ alkoxy,
which process comprises the reaction of a compound (III): 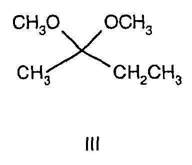 with an acylating agent, in the presence of pyridine, of the formula (IV): 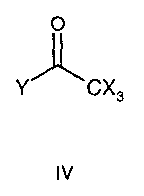 wherein X and Y independently represent Cl or F, optionally in the presence of an activating agent, followed, after between about 8 and 24 hours by addition of a hydrazine compound of general formula (V): 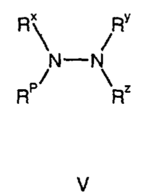 wherein when R^{P} represents H, then
R^{x} = EWG, R^{y} and R^{z} = H; or
R^{x} and R^{y} = H and R^{z} = EDG; or
R^{x} = R^{y} = R^{z} = H when R¹ = EWG; or
R^{x} = EWG, R^{y} = H and R^{z} = EDG wherein EDG is a tri(C₁-C₂)alkylsilyl group and wherein EWG is selected from *tert*-butyloxycarbonyl and trifluoroacetamide.
and wherein when R^{P} represents R¹ wherein R¹ is as defined hereinbefore, then
R^{x} = H, R^{y} = H and R^{z} = EWG; or
R^{x} = EDG, R^{y} = H and R^{z} = H; or
R^{x} = R^{y} = R^{z} = H when R¹ = EDG; or
R^{x} = EDG, R^{y} = H and R^{z} = EWG wherein EDG is a tri(C₁-C₂)alkylsilyl group and wherein EWG is selected from *tert*-butyloxycarbonyl and trifluoroacetamide.

The process of the invention may be carried out in accordance with reaction conditions known to those skilled in the art

The process according to the present invention requires an activating agent to "activate" the compound of general formula (III) and at least one equivalent of an acylating agent of general formula (IV) to "react" with the activated compound generated from the compound of general formula (III). Any suitable agent capable of activating the compound of general formula (III), may be used in conjunction with at least one equivalent of acylating agent of general formula (IV) according to the process of the present invention.

The activating agent should be capable of converting an acetal to an enol ether under the basic reaction conditions. Suitable activating agents could be, trialkylsilyl halides, trialkylsilyl trifluoromethanesulfonates, oxalyl halides, 2-(trifluoroacetoxy)pyridine, 1-(trifluoroacetyl)imidazole, trifluoroacetyl chloride, trifluoroacetic anhydride, tribromoacetyl chloride and trichloroacetyl chloride.

More preferable activators are 2-(trifluoroacetoxy)pyridine, 1-(trifluoroacetyl)imidazole, trifluoroacetyl chloride, triflouroacetic anhydride, tribromoacetyl chloride and trichloroacetyl chloride.

In a preferred process according to the present invention the acylating agent of general formula (IV) can be used as both the activating agent and the acylating agent.
The acylating agent (IV) must be capable of acylating an enol ether to afford the key enone intermediate. Suitable reagents include be 2-(trifluoroacetoxy)pyridine, 1-(trifluoroacetyl) imidazole, trifluoroacetyl chloride, trifluoroacetic anhydride, tribromoacetyl chloride and trichloroacetyl chloride. Preferred for use herein are trifluoroacetic anhydride and trichloroacetyl chloride. Even more preferred for use herein is trichloroacetylchloride.

Thus according to a yet further aspect of the present invention there is provided a process for the production of pyrazole compounds of general formula (II), as defined hereinbefore, which process comprises the reaction of a compound (III), as defined herein before, with at least one equivalent, more preferably at least two equivalents of an acylating agent of the formula (IV), as defined hereinbefore, optionally in the presence of an activating agent, followed by reaction with a hydrazine compound of general formula (V), as defined hereinbefore.

The reaction between the compound of the general formula (III) and the activating agent (and/or the acylating agent of general formula (IV), for reactions wherein the activating and acylating agents are the same) according to the process of the present invention may be carried out in an appropriate organic solvent system, which solvent system should not significantly react chemically with, or significantly give rise to stereochemical changes in, the reactants or product once formed, or significantly give rise to other side reactions. Suitable solvents include: halogenated hydrocarbons (such as chloroform, dichloromethane and 1,2-dichloroethane), ethers (such as tetrahydrofuran, 1,4-dioxan, diethyl ether and *tert*-butyl methyl ether), aromatic hydrocarbons (such as toluene, xylenes and chlorobenzene) and alkyl acetates (such as ethyl acetate) and mixtures thereof. A preferred solvent is dichloromethane.

The reaction between the compound of the general formula (III) and the activating agent (and/or acylating agent of general formula (IV)) according to the process of the present invention may be carried at from O°C to about room temperature, and, preferably, in an inert atmosphere (i.e. in the presence of an inert gas, such as nitrogen or argon).

Following the activation and acylation of the compound of general formula (III) the hydrazine compound of general formula (V) is added directly to the reaction mixture (of the activating agent (and/or acylating agent of general formula (IV)) and the compound of general formula (III)) *in situ* to provide a compound of general formula (II) according to the process of the present invention. The hydrazine compound (V) may be added portionwise, dropwise, in solution or neat. Typically the hydrazine compound is added in water and/or a suitable organic solvent (e.g. alcohols such as methanol, ethanol or *iso*-propanol, to provide a compound of general formula (II) wherein R³ is C₁ to C₆ alkoxy), or aqueous ammonia (to provide a compound of general formula (II) wherein R³ is NR⁴R⁵) or mixtures thereof, followed by removal of the original reaction solvent (e.g. dichloromethane) and heat treatment.

In reactions with hydrazine compounds of general formula (V) wherein R^{P} = R¹ and wherein R¹ is an acid labile group such as a tent-butyloxycarbonyl group, the pH of the reaction mixture may be adjusted to between about pH 1.5 and about pH 3 and preferably to about pH 2 following addition of the hydrazine compound.

In reactions with hydrazine compounds of general formula (V) wherein R^{P} = H, it is not necessary to adjust the pH of the reaction mixture following addition of the hydrazine compound.

In a yet further aspect of the process according to the present invention compounds of general formula (II) wherein R³ = OH may be prepared either via conversion of pyrazole compounds of general formula (II) wherein R³ = alkoxy as obtained according to the process described hereinbefore, or, alternatively via an *in situ* conversion (of the ester to the acid) wherein the pH of the reaction mixture is raised to greater than about pH 8, via addition of a suitable base such as for example NaOH.

In a yet further aspect of the process according to the present invention compounds of general formula (II) prepared as outlined hereinbefore via (III), (IV) and (V) using aqueous ammonia as solvent i.e. a compound of general formula (II) wherein R³ is NR⁴R⁵ may be converted directly to provide a compound of the general formula B (as illustrated in Scheme 1).

Appropriate reaction times and reaction temperatures depend upon the solvent system that is employed, as well as the compound that is to be formed, but these may be determined routinely by the skilled person.

Compounds of general formula (III) when not commercially available may be prepared by known techniques as detailed in the preparations section herein.

Compounds of general formulae (IV) and (V), and derivatives thereof, when not commercially available or not subsequently described, may be obtained by conventional synthetic procedures or by analogy with the processes described herein, in accordance with standard techniques, from readily available starting materials using appropriate reagents and reaction conditions.

According to a further aspect of the present invention the compounds of general formula general formula I: 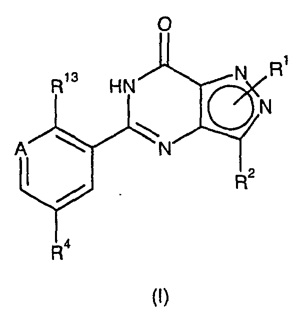 or a pharmaceutically or veterinarily acceptable salt thereof, or a pharmaceutically or veterinarily acceptable solvate of either entity, wherein:
A is CH or N;
R¹ is H, C₁ to C₆ alkyl, C₃ to C₆ alkenyl, C₃ to C₆ cycloalkyl, C₃ to C₆ cycloalkenyl, or C₁-C₃ perfluoroalkyl, wherein said alkyl group may be branched or straight chain and wherein said alkyl, alkenyl, cycloalkyl or perfluoroalkyl group is optionally substituted by; one or more substituents selected from: hydroxy; C₁ to C₄ alkoxy; C₃ to C₆ cycloalkyl; C₁-C₃ perfluoroalkyl; phenyl substituted with one or more substitutents selected from C₁ to C₃ alkyl, C₁ to C₄ alkoxy, C₁ to C₄ haloalkyl or C₁ to C₄ haloalkoxy wherein said haloalkyl and haloalkoxy groups contain one or more halo atoms, halo, CN, NO₂, NHR¹¹, NHSO₂R¹², SO₂R¹², SO₂NHR¹¹, COR¹¹, CO₂R¹¹ wherein R¹¹ is H, C₁ to C₄ alkyl, C₂ to C₄ alkenyl, C₁ to C₄ alkanoyl, C₁ to C₄ haloalkyl or C₁ to C₄ haloalkoxy and wherein R¹² is C₁ to C₄ alkyl, C₂ to C₄ alkenyl, C₁ to C₄ alkanoyl, C₁ to C₄ haloalkyl or C₁ to C₄ haloalkoxy; NR⁷R⁸, CONR⁷R⁸ or NR⁷COR¹¹ wherein R⁷ and R⁸ are each independently selected from H, C₁ to C₄ alkyl, C₂ to C₄ alkenyl, C₁ to C₄ alkoxy, CO₂R⁹, SO₂R⁹ wherein said alkyl, alkenyl or alkoxy groups are optionally substituted by NR⁵R⁶, C₁ to C₄ haloalkyl or C₁ to C₄ haloalkoxy and wherein R⁹ is H, hydroxy C₂ to C₃ alkyl, C₁ to C₄ alkanoyl or C₁ to C₄ alkyl which is optionally substituted with phenyl wherein said phenyl group is optionally substituted by one or more substituents selected from C₁ to C₄ alkyl optionally substituted by C₁ to C₄ haloalkyl or C₁ to C₄ haloalkoxy, C₁ to C₄ alkoxy, halo, CN, NO₂, NHR¹¹, NHSO₂R¹², SO₂R¹², SO₂NHR¹¹, COR¹¹ or CO₂R¹¹; Het¹; Het² or Het³; or R¹ is Het⁴ or phenyl wherein said phenyl group is optionally substituted by one or more substituents selected from C₁ to C₄ alkyl, C₂ to C₄ alkenyl, C₁ to C₄ alkoxy, halo, CN, CF₃, OCF₃, NO₂, NHR¹¹, NHSO₂R¹², SO₂R¹², SO₂NHR¹¹, COR¹¹, CO₂R¹¹;
R² is H, C₁ to C₆ alkyl, C₃ to C₆ alkenyl or (CH₂)ₙ(C₃ to C₆ cycloalkyl) wherein n is 0, 1 or 2 and wherein said alkyl or alkyenyl group is optionally substituted with one or more fluoro substituents;
R¹³ is OR³ or NR⁵R⁶;
R³ is C₁ to C₆ alkyl, C₃-C₆ alkenyl, C₃-C₆ alkynyl, C₃-C₇ cycloalkyl, C₁-C₆ perfluoroalkyl or (C₃-C₆ cycloalkyl)C₁-C₆ alkyl optionally substituted with one or two substituents selected from C₃ to C₅ cycloalkyl, hydroxy, C₁ to C₄ alkoxy, C₃-C₆ alkenyl, C₃-C₆ alkynyl, benzyloxy, NR⁵R⁶, phenyl, Het¹, Het², Het³ or Het⁴ wherein the C₁ to C₆ alkyl and C₁ to C₄ alkoxy groups may optionally be terminated by a haloalkyl group such as CF₃; C₃ to C₆ cycloalkyl; Het¹, Het², Het³ or Het⁴;
R⁴ is C₁-C₄ alkyl optionally substituted with OH, NR⁵R⁶, CN, CONR⁵R⁶ or CO₂R⁷; C₂-C₄ alkenyl optionally substituted with CN, CONR⁵R⁶ or CO₂R⁷; C₂-C₄ alkanoyl optionally substituted with NR⁵R⁶; hydroxy C₂-C₄ alkyl optionally substituted with NR⁵R⁶; (C₂-C₃ alkoxy)C₁-C₂ alkyl optionally substituted with OH or NR⁵R⁶; CONR⁵R⁶; CO₂R⁷; halo; NR⁵R⁶; NHSO₂NR⁵R⁶; NHSO₂R⁸; or phenyl or heterocyclyl either of which is optionally substituted with methyl; or R⁴ is a pyrrolidinylsulphonyl, piperidinosulphonyl, morpholinosulphonyl, or piperazin-1-ylsulphonyl group having a substituent, R¹⁰ at the 4-position of the piperazinyl group wherein said piperazinyl group is optionally substituted with one or two C₁ to C₄ alkyl, C₁ to C₃ alkoxy, NR⁷R⁸ or CON R⁷R⁸ groups and is optionally in the form of its 4-N-oxide;
R⁵ and R⁶ are each independently selected from H and C₁ to C₄ alkyl optionally substituted with C₃ to C₅ cycloalkyl or C₁ to C₄ alkoxy, or, together with the nitrogen atom to which they are attached, form an azetidinyl, pyrrolidinyl, piperidinyl, morpholinyl, 4-(NR⁹)- piperazinyl or imidazolyl group wherein said group is optionally substituted with methyl or hydroxy;
R¹⁰ is H; C₁ to C₆ alkyl, (C₁-C₃ alkoxy) C₂-C₆ alkyl, hydroxy C₂-C₆ alkyl, (R⁷R⁸N)C₂-C₆ alkyl, (R⁷R⁸NCO)C₁-C₆ alkyl, CONR⁷R⁸, CSNR⁷R⁸ or C(NH)NR⁷R⁸ optionally substituted with one or two substituents selected from hydroxy, NR⁵R⁶, CONR⁵R⁶, phenyl optionally substituted with C₁ to C₄ alkyl or C₁ to C₄ alkoxy; C₂ to C₆ alkenyl or Het⁴;
Het¹ is an N-linked 4-, 5- or 6-membered nitrogen-containing heterocyclic group optionally containing one or more further heteroatoms selected from S, N or O;
Het² is a C-linked 5-membered heterocyclic group containing an O, S or N heteroatom optionally containing one or more heteroatoms selected from O or S;
Het³ is a C-linked 6-membered heterocyclic group containing an O or S heteroatom optionally containing one or more heteroatoms selected from O, S or N or Het³ is a C-linked 6-membered heterocyclic group containing three N heteroatoms;
Het⁴ is a C-linked 4-, 5- or 6-membered heterocyclic group containing one, two or three heteroatoms selected from S, O or N; and wherein any of said heterocyclic groups Het¹, Het², Het³ or Het⁴ may be saturated, partially unsaturated or aromatic and wherein any of said heterocyclic groups may be optionally substituted with one or more substituents selected from C₁ to C₄ alkyl, C₂ to C₄ alkenyl, C₁ to C₄ alkoxy, halo, CO₂R¹¹, COR¹¹, SO₂R¹² or NHR¹¹ and/or wherein any of said heterocyclic groups is benzo-fused;
or wherein when R¹³ represents OR³ or R³NR⁵; R¹ represents Het, alkylHet, aryl or alkylaryl, which latter five groups are all optionally substituted and/or terminated with one or more substituents selected from halo, cyano, nitro, C₁-C₆ alkyl, halo(C₁-C₆ alkyl), OR⁶, OC(O)R⁷, C(O)R⁸, C(O)OR⁹, C(O)NR¹⁰R¹¹, NR¹²R¹³ and SO₂NR¹⁴R¹⁵; R² represents H, halo, cyano, nitro, OR⁶, OC(O)R⁷, C(O)R⁸, C(O)OR⁹, C(O)NR¹⁰R¹¹, NR¹²R¹³, SO₂NR¹⁴R¹⁵, C₁-C₆ alkyl, Het, alkylHet, aryl or alkylaryl, which latter five groups are all optionally substituted and/or terminated with one or more substituents selected from halo, cyano, nitro, C₁-C₆ alkyl, halo(C₁-C₆ alkyl), OR⁶, OC(O)R⁷, C(O)R⁸, C(O)OR⁹, C(O)NR¹⁰R¹¹, NR¹²R¹³ and SO₂NR¹⁴R¹⁵; R³ represents H, C₁-C₆ alkyl, alkylHet or alkylaryl, which latter three groups are all optionally substituted and/or terminated with one or more substituents selected from halo, cyano, nitro, C₁-C₆ alkyl, halo(C₁-C₆ alkyl), OR⁶, OC(O)R⁷, C(O)R⁸, C(O)OR⁹, C(O)NR¹⁰R¹¹, NR¹²R¹³ and SO₂NR¹⁴R¹⁵; R⁴ represents H, halo, cyano, nitro, halo(C₁-C₆ alkyl), OR⁶, OC(O)R⁷, C(O)R⁸, C(O)OR⁹, C(O)NR¹⁰R¹¹, NR¹²R¹³, NR¹⁶Y(O)R¹⁷, SOR¹⁸, SO₂R¹⁹R²⁰, C(O)AZ, C₁-C₆ alkyl, C₂-C₆ alkenyl, C₂-C₆ alkynyl, Het, alkylHet, aryl, alkylaryl, which latter seven groups are all optionally substituted and/or terminated with one or more substituents selected from halo, cyano, nitro, C₁-C₆ alkyl, halo(C₁-C₆ alkyl), OR⁶, OC(O)R⁷, C(O)R⁸, C(O)OR⁹, C(O)NR¹⁰R¹¹, NR¹²R¹³ and SO₂NR¹⁴R¹⁵; Y represents C or S(O), wherein one of R¹⁶ and R¹⁷ is not present when Y is S(O); A represents C₂-C₆ alkylene; Z represents OR⁶, halo, Het or aryl, which latter two groups are both optionally substituted with one or more substituents selected from halo, cyano, nitro, C₁-C₆ alkyl, halo(C₁-C₆ alkyl), OR⁶, OC(O)R⁷, C(O)R⁸, C(O)OR⁹, C(O)NR¹⁰R¹¹, NR¹²R¹³ and SO₂NR¹⁴R¹⁵; R⁵, R⁶, R⁷, R⁸, R⁹, R¹⁸, R¹⁹ and R²⁰ independently represent H or C₁-C₆ alkyl; R¹⁰ and R¹¹ independently represent H or C₁-C₆ alkyl, which latter group is optionally substituted and/or terminated with one or more substituents selected from halo, cyano, nitro, C₁-C₆ alkyl, halo(C₁-C₆ alkyl), OR⁶, OC(O)R⁷, C(O)R⁸, C(O)OR⁹, C(O)NR¹⁰R¹¹, NR¹²R¹³ and SO₂NR¹⁴R¹⁵ or Het or aryl optionally substituted with one or more of said latter eleven groups or one of R¹⁰ and R¹¹ may be C₁-C₆ alkoxy, amino or Het, which latter two groups are both optionally substituted with C₁-C₆ alkyl; R¹² and R¹³ independently represent H or C₁-C₆ alkyl or one of R¹² or R¹³ may be C(O)- C₁-C₆ alkyl or C(O)Het in which Het is optionally substituted with C₁-C₆ alkyl; R¹⁴ and R¹⁵ independently represent H or C₁-C₆ alkyl or R¹⁴ and R¹⁵, together with the nitrogen atom to which they are bound, form a heterocyclic ring; R¹⁶ and R¹⁷ independently represent H or C₁-C₆ alkyl or one of R¹⁶ and R¹⁷ may be Het or aryl, which latter two groups are both optionally substituted with C₁-C₆ alkyl; Het represents an optionally substituted four to twelve membered heterocyclic group, which may be aromatic or non-aromatic, which may contain one or more double bonds, which may be mono- or bi-cyclic and which contains one or more heteroatoms selected from N, S and O;
wherein said compounds may be prepared from compounds of general formula (VIII): 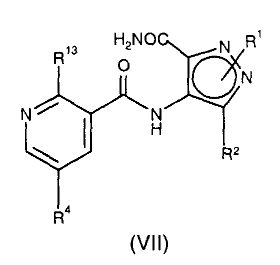 wherein R¹, R², R⁴ and R¹³ are as defined hereinbefore wherein said compound of general formula (VIII) is prepared from the reaction of a compound of general formula (VII): 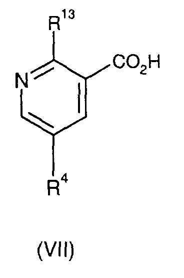 wherein R⁴ and R¹³ are as defined herein before via coupling with a compound of general formula (VI): 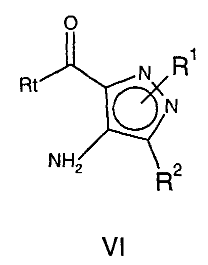 wherein R¹ and R² are as defined hereinbefore and wherein R^{t} is NR^{p}R^{q}
wherein R^{p} and R^{q} independently represent H or C₁-C₆ alkyl and wherein said compound of general formula (VI) is prepared by nitration and hydrogenation of a compound of general formula (II): 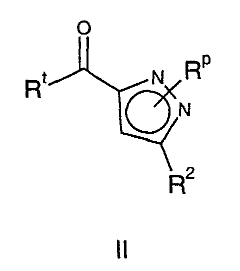 wherein R^{t} and R² are as defined hereinbefore and wherein R^{P} represents R¹ as defined hereinbefore
characterised in that said compound of general formula (II) is prepared from the reaction of a compound of general formula (III), 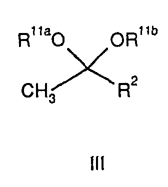 wherein R^{11a} and R^{11b} independently represent C₁-C₆ alkyl, R² is as defined herein before,
with an acylating agent of the formula (IV) in the presence of a base, 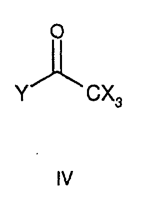 wherein X represents halogen independently selected from Cl, F or Br, and Y represents halogen or OR¹² wherein R¹² is: C₁-C₆ alkyl; C(O)CX₃; Het; or C₀-C₆ alkyl(Het) wherein Het is pyridine or imidazole,
followed by *in situ* reaction with a hydrazine compound of general formula (V) 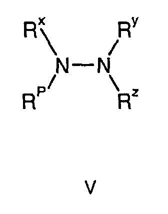 R^{P} represents H or R¹ wherein R¹ is as defined hereinbefore, and R^{x}, R^{y} and R^{z} are independently selected from H or electron donating groups (EDG) or electron withdrawing groups (EWG) wherein said electron withdrawing or donating groups are labile under the conditions of the reaction,
wherein said process for the preparation of compound (II) is optionally carried out in the presence of an activating agent.

According to a preferred process of the present invention compounds of formula (I) are prepared from compounds of formula (II) wherein R¹ represents: H, C₁-C₆ alkyl; C₁-C₆ alkoxy; C₃-C₆ cycloalkyl; C₁-C₆ alkyl(C₁-C₆ alkoxy), Het, C₁-C₆ alkylHet, aryl or C₁-C₆ alkylaryl, which latter eight groups are all optionally substituted (and/or, in the case of C₁-C₆ alkyl, optionally terminated) by one or more substituents selected from halo, cyano, nitro, C₁-C₆ alkyl, C(O)NR⁴R⁵, C(O)R⁶, C(O)OR⁷, OR⁸, NR^{9a}R^{9b} and SO₂NR^{10a}R^{10b};
R² represents C₁-C₆ alkyl; C₁-C₆ alkoxy; C₃-C₆ cycloalkyl; C₁-C₆ alkyl(C₁-C₆ alkoxy), Het, C₁-C₆ alkylHet, aryl or C₁-C₆ alkylaryl, which latter eight groups are all optionally substituted (and/or, in the case of C₁-C₆ alkyl, optionally terminated) by one or more substituents selected from halo, cyano, nitro, C₁-C₆ alkyl, C(O)NR⁴R⁵, C(O)R⁶, C(O)OR⁷, OR⁸, NR^{9a}R^{9b} and SO₂NR^{10a}R^{10b};
R^{t} represents: NR^{p}R^{q};
R^{p}, R^{q}, R⁶, R⁷, R⁸, R^{10a} and R^{10b} independently represent H or C₁-C₆ alkyl;
R^{9a} and R^{9b} either independently represent, H or C₁-C₆ alkyl or, together with the nitrogen atom to which they are attached, represent azetidinyl, pyrollidinyl or piperidinyl;
R⁴ is C₁-C₄ alkyl optionally substituted with OH, NR⁵R⁶, CN, CONR⁵R⁶ or CO₂R⁷; or R⁴ is CO₂R⁷; or R⁴ is a pyrrolidinylsulphonyl, piperidinosulphonyl, morpholinosulphonyl, or piperazin-1-ylsulphonyl group having a substituent, R¹⁰ at the 4-position of the piperazinyl group wherein said piperazinyl group is optionally substituted with one or two C₁ to C₄ alkyl, C₁ to C₃ alkoxy, NR⁷R⁸ or CON R⁷R⁸ groups and is optionally in the form of its 4-N-oxide;
R¹⁰ represents H or C₁-C₆ alkyl.

Highly preferred herein is a process for the preparation of compounds of general formula (I) from compounds of general formula (II) wherein R¹ represents C₁₋₄ alkyl, which alkyl group is optionally interrupted by an oxygen atom, and/or is optionally terminated by a Het group (such as a pyridinyl group);
R² represents C₁₋₄ alkyl;
R³ represents C₁₋₅ alkyl, which alkyl group is optionally interrupted by an oxygen atom;
R⁴ is CO₂R⁷; or R⁴ is a morpholinosulphonyl, or piperazin-1-ylsulphonyl group having a substituent, R¹⁰ at the 4-position of the piperazinyl group wherein R¹⁰ represents H, methyl or ethyl.

More preferred compounds of formulae I, IA and IB prepared according to a process of the invention include those in which:
R¹ represents linear C₁₋₃ alkyl, which alkyl group is optionally interrupted by an oxygen atom, or is optionally terminated by a 2-pyridinyl group (e.g. to form a 2-pyridinylmethyl group);
R² represents linear C₂₋₃ alkyl;
R³ represents linear or branched C₂₋₄ alkyl, which alkyl group is optionally interrupted by an oxygen atom;
R⁴ is CO₂R⁷; or R⁴ is a morpholinosulphonyl, or piperazin-1-ylsulphonyl group having a substituent, R¹⁰ at the 4-position of the piperazinyl group wherein R¹⁰ represents methyl or ethyl.

Particularly preferred compounds that may be formed according to a process of the invention include sildenafil (1A), and the following five compounds:

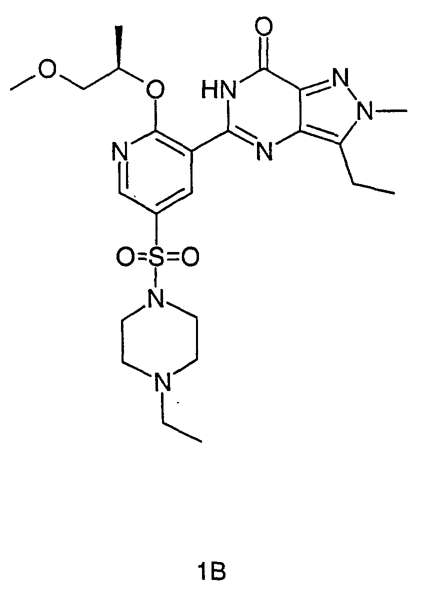

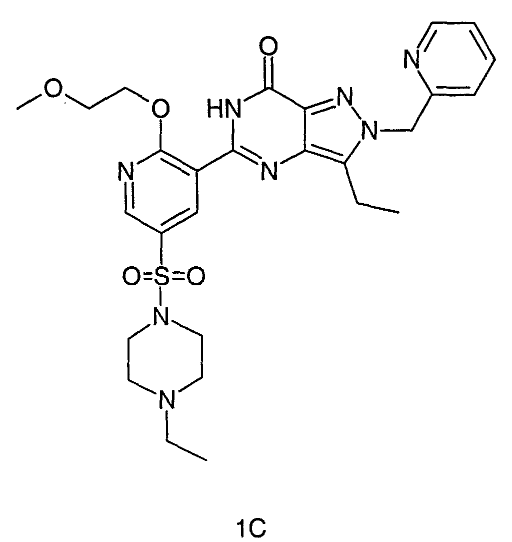

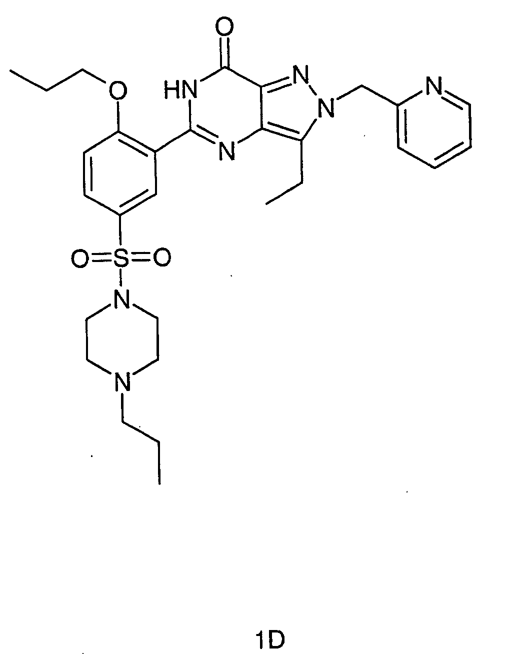

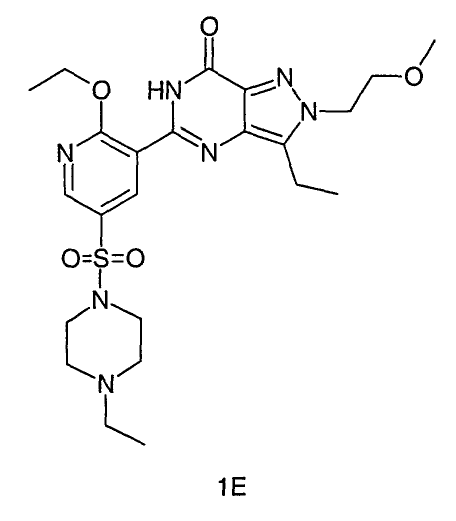

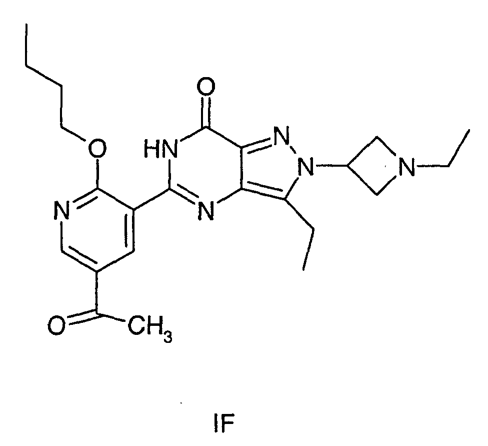

Said compounds 1B, 1C, 1D, 1E and 1F are otherwise known as: 1B, (+)-3-ethyl-5-[5-(4-ethylpiperazin-1-ylsulphonyl)-2-(2-methoxy-1(R)-methylethoxy)pyridin-3-yl]-2-methyl-2,6-dihydro-7H-pyrazolo[4,3-d]pyrimidin-7-one also known as 3-Ethyl-5-{5-[4-ethylpiperazin-1-ylsulphonyl]-2-([(1 R)-2-methoxy-1-methylethyl]oxy)pyridin-3-yl}-2-methyl-2,6-dihydro-7H-pyrazolo[4,3-d] pyrimidin-7-one, the compound of Example 118 of WO99/54333; 1C, 3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrimidin-7-one, the compound of Example 5 of WO98/49166; 1D, 3-ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d]pyrimidin-7-one, the compound of Example 4 of WO99/54333; 1E, 5-[2-Ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one, also known as 1-{6-ethoxy-5-[3-ethyl-6,7-dihydro-2-(2-methoxyethyl)-7-oxo-2*H*-pyrazolo[4,3-*d*]pyrimidin-5-yl]-3-pyridyl sulphonyl}-4-ethylpiperazine, the compound of Example 103 of WO 01/27113, exemplified hereinafter as Example 1; 1F, 5-(5-acetyl-2-butoxy-3-pyridinyl)-3-ethyl-2-(1-ethyl-2-(1-ethyl-3-azetidinyl)-2,6-dihydro-*7H*-pyrazolo[4,3-*d*]pyrimidin-7-one, the compound of Example 132 of WO 01/27112, exemplified hereinafter as Example 2.

Compounds may be isolated from reaction mixtures using known techniques.

Substituents on the aryl (e.g. phenyl), and (if appropriate) heterocyclic, group(s) in compounds defined herein may be converted to other substituents using techniques well known to those skilled in the art. For example, amino may be converted to amido, amido may be hydrolysed to amino, hydroxy may be converted to alkoxy, alkoxy may be hydrolysed to hydroxy etc.

It will be appreciated by those skilled in the art that, in the processes described above, the functional groups of intermediate compounds may be, or may need to be, protected by protecting groups.

Functional groups which it is desirable to protect thus include hydroxy, amino and carboxylic acid. Suitable protecting groups for hydroxy include trialkylsilyl and diarylalkylsilyl groups (e.g. *tert*-butyldimethylsilyl, *tert*-butyldiphenylsilyl or trimethylsilyl), tetrahydropyranyl, benzyl and alkylcarbonyl groups (e.g. methyl- and ethylcarbonyl groups). Suitable protecting groups for amino include benzyl, *tert*-butyloxycarbonyl, 9-fluorenylmethoxycarbonyl or benzyloxycarbonyl. Suitable protecting groups for carboxylic acid include C₁₋₆ alkyl, allyl or benzyl esters.

The protection and deprotection of functional groups may take place before or after any of the reaction steps described hereinbefore.

Protecting groups may be removed in accordance with techniques which are well known to those skilled in the art and as described hereinafter.

The use of protecting groups is fully described in "Protective Groups in Organic Chemistry", edited by JWF McOmie, Plenum Press (1973), and "Protective Groups in Organic Synthesis", 3^{rd} edition, TW Greene & PGM Wutz, Wiley-Interscience (1999).

The process of the invention possesses the advantage that the intermediate pyrazoles of general formula (II) which are used in the synthesis of pyrimidin-7-ones, and in particular in the preparation of sildenafil, compound IA herein, may be prepared from commercially-available starting materials in fewer steps than in processes described in the prior art, without concomitant losses in terms of yield of key intermediates and of final compounds. Further, said pyrazoles are obtained in desirable levels of purity according to the process of the present invention.

Further, the process of the invention may have the advantage that pyrazole compounds of general formula (II) may be prepared in less time, more conveniently, and at a lower cost, than when prepared in processes described in the prior art.

The invention is illustrated, but in no way limited, by the following examples.

All ¹H NMR spectra were recorded using a Varian Unity 300 MHz machine.

### Preparation 1

### 2,2-dimethoxybutane

Methyl ethyl ketone (672 mL) was charged to a 2L round bottomed flask and stirred at room temperature before being treated with, trimethylorthoformate (763 mL) and para-toluenesulphonic acid (6.65 g, 0.5 mol%). Over a 15 min period the internal temperature rose to 46°C, so the reaction was cooled to 0°C for 30 min. The reaction was then stirred at room temperature for 2 h. The reaction was then neutralised by pouring onto sodium carbonate (ca. 750 g) with constant stirring. The resultant slurry was filtered under vacuum and the resultant filtrate was distilled at atmospheric pressure. The fraction boiling in the range 118°C-124°C was collected as a colourless liquid, 582 g, 70%.
¹H NMR (CDCl₃): δ = 0.88 (3H, t), 1.24 (3H, s), 1.61 (2H, q), 3.17 (6H, s).

### Example 1

### N-[3-Carbamoyl-5-ethyl-1-(2-methoxyethyl)-1H-pyrazol-4-yl}-2-ethoxy-5-(4-ethyl-1-piperazinyl sulfonyl) nicotinamide.

### (a) Ethyl 3-ethyl-1H-pyrazole-5-carboxylate (IIA) from (III) and (V)

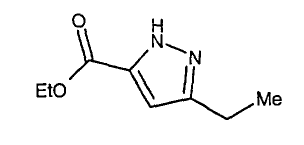

To a stirred solution of 2,2-dimethoxybutane (10 g, 84.7 mMol) in CH₂Cl₂ (50 mL) under a nitrogen atmosphere at 0°C was added pyridine (13.7 mL, 169.5 mMol). The reaction mixture was maintained at 0°C and a solution of trichloroacetyl chloride (18.9 mL, 169.5 mMol) in CH₂CL₂ (35 mL) was added over 1 hour with constant stirring. The yellow-orange solution begins to precipitate a white solid as the reaction progresses. The reaction mixture is allowed to warm to room temperature over 20 h. The reaction mixture was diluted with ethanol (150 mL) and re-cooled to 0°C before treatment with hydrazine hydrate (8.2 mL, 169.5 mMol) as a solution in ethanol (35 mL) over 30 min. The reaction was heated to 50°C and solvent was distilled at atmospheric pressure. The temperature was increased until the head temperature reached 78°C. Reflux was maintained for a further 2 h, before cooling to room temperature. The reaction mixture was diluted with water (250 mL) and ethanol was removed by evaporation at reduced pressure. The resultant mixture was extracted with CH₂Cl₂ (3 x 200 mL). The combined organics were dried (MgSO₄), filtered and evaporated at reduced pressure to afford the title compound as a brown oil, 12.05 g, 85%.
¹H NMR (300 MHz, CDCl₃): δ=1.20 (3H, t), 1.28 (3H, t), 2.67 (2H, q), 4.29 (2H, q), 6.55 (1H, s), 12.56 (1H, s).

LRMS *m*/*z*= 167.1 [M-H]⁺, C₈H₁₂N₂O₂ requires 168.2.

### (b) Ethyl 3-ethyl-1H-pyrazole-5-carboxvlic acid (IIA) from (IIA) via route 1

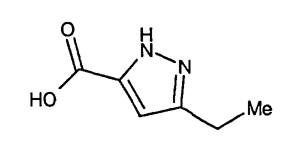

Aqueous sodium hydroxide solution (10M; 100 ml, 1.0 mol) was added dropwise to a stirred suspension of the title compound of Example (a) (66.0 g, 0.39 mol) in methanol and the resulting solution heated under reflux for 4 hours. The cool reaction mixture was concentrated under reduced pressure to ca. 200 ml, diluted with water (200 ml) and this mixture washed with toluene (3 x 100 ml). The resulting aqueous phase was acidified with concentrated hydrochloric acid to pH 4 and the white precipitate collected and dried by suction to provide the title compound (34.1 g). δ (DMSO_{d6}): 1.13 (3H,t), 2.56 (2H,q), 6.42 (1H,s).

### (c) 4-Nitro-3-n-propyl-1H-pyrazole-5-carboxylic acid

Fuming sulphuric acid (17.8 ml) was added dropwise to stirred, ice-cooled fuming nitric acid (16.0 ml), the resulting solution heated to 50°C, then 3-n-propyl-1 H-pyrazole-5-carboxylic acid (Chem. Pharm. Bull., 1984, 32, 1568; 16.4 g, 0.106 mol) added portionwise over 30 minutes whilst maintaining the reaction temperature below 60°C. The resulting solution was heated for 18 hours at 60°C, allowed to cool, then poured onto ice. The white precipitate was collected, washed with water and dried by suction to yield the title compound (15.4 g), m.p. 170-172°C. Found: C, 42.35; H, 4.56; N, 21.07. C₇H₉N₃O₄ requires C, 42.21; H, 4.55; N, 21.10%. δ (DMSO_{d6}): 0.90 (3H,t), 1.64 (2H,m), 2.83 (2H,m), 14.00 (1H,s).

### (d) 3-Ethyl-4-nitro-1H-pyrazole-5-carboxylic acid (IIA) to (AA) via route 2

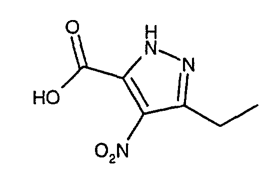

Obtained from the title compound of Example (b), by analogy with the process of Example (c), as a brown solid (64%). δ (DMSO_{d6}): 1.18 (3H,t), 2.84 (2H,m), 13.72 (1H,s).

### (e) 4-Nitro-3-n-propyl-1H-pyrazole-5-carboxamide

A solution of the title compound of Example (c) (15.4 g, 0.077 mol) in thionyl chloride (75 ml) was heated under reflux for 3 hours and then the cool reaction mixture evaporated under reduced pressure. The residue was azeotroped with tetrahydrofuran (2 x 50 ml) and subsequently suspended in tetrahydrofuran (50 ml), then the stirred suspension ice-cooled and treated with gaseous ammonia for 1 hour. Water (50 ml) was added and the resulting mixture evaporated under reduced pressure to give a solid which, after trituration with water and drying by suction, furnished the title compound
(14.3 g), m.p. 197-199°C. Found: C, 42.35; H, 5.07; N, 28.38. C₇H₁₀N₄O₃ requires C, 42.42; H, 5.09; N, 28.27%. δ (DMSO_{d6}): 0.90 (3H,t), 1.68 (2H,m), 2.86 (2H,t), 7.68 (1H,s), 8.00 (1H,s).

### (f) 3-Ethyl-4-nitro-1H-pyrazole-5-carboxamide BA from AA via route 3

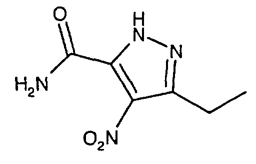

Obtained from the title compound of Example (d), by analogy with Example (e), as a white solid (90%). δ (DMSO_{d6}): 1.17 (3H,t), 2.87 (2H,m), 7.40 (1H,s), 7.60 (1H,s), 7.90 (1H,s). LRMS: m/z 185 (M+1)⁺.

### (g)(i)5-Ethyl-1-(2-methoxyethyl)-4-nitro-1H-pyrazole-3-carboxamide CA from BA via route 4

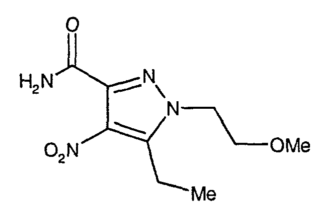

A mixture of 3-ethyl-4-nitro-1*H*-pyrazole-5-carboxamide (2.5 kg, 13.6 Mol), sodium carbonate (1.8 Kg, 17.0 Mol) and 2-bromoethyl methyl ether (1.98 kg, 14.2 Mol) in THF (22.5 L) and water (2.5 L) was heated under reflux and stirred for 20 hours. The mixture was cooled to ambient temperature and CH₂Cl₂ (67.5 L) and water (22.5 L) were added. The resultant organic and aqueous layers were separated. The aqueous phase was extracted with CH₂Cl₂ (22.5 L) and the combined organic solution was distilled under atmospheric pressure and replaced with ethyl acetate (33 L) to a final volume of 17 L. The cooled mixture was granulated at ambient temperature for 2 hours, filtered and washed with ethyl acetate (2.5 L). This afforded 5-ethyl-1-(2-methoxyethyl)-4-nitro-1*H*-pyrazole-3-carboxamide as a white crystalline solid, 2.10 kg, 57%. m.p. = 140°C. Found: C, 44.46; H, 5.79; N, 23.01. C₉H₁₄N₄O₄ requires C, 44.63; H, 5.79; N, 23.14%.
δ(CDCl₃): 1.18 (3H, t), 2.98 (2H, q), 3.22 (3H, s), 3.77 (2H, t), 4.28 (2H, q), 6.03 (1H, s), 7.36 (1H, s).
LRMS: m/z = 243 (M+1)⁺

### (g)(ii) 5-Ethyl-1-(2-methoxyethyl)-4-nitro-1H-pyrazole-3-carboxamide.

A mixture of 3-ethyl-4-nitro-*1H*-pyrazole-5-carboxamide (25 g, 0.136 Mol), sodium carbonate (18 g, 0.17 Mol) and sodium iodide (20.4 g, 0.136 Mol) were suspended in ethyl methyl ketone (125 mL) at room temperature. 2-bromoethyl methyl ether (12.8 mL, 0.142 Mol) was added and the mixture was heated to reflux and stirred for 70 hours. The mixture was cooled to ambient temperature and water (250 mL) was added. The resultant slurry was warmed to reflux and held at that temperature for 30 min before cooling to room temperature. The resultant precipitate was granulated at room temperature for 3 h, filtered and vacuum dried to afford 5-ethyl-1-(2-methoxyethyl)-4-nitro-1H-pyrazole-3-carboxamide as a yellow crystalline solid 24.3 g, 74%. Data as reported for Example (g)(i).

### (h) 4-Amino-5-ethyl-1-(2-methoxyethyl)-1H-pyrazole-3-carboxamide (IA) from CA via route 5

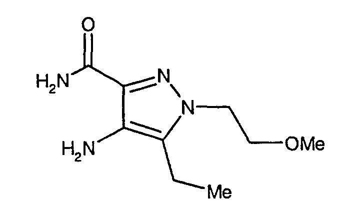

A mixture of 5-ethyl-1-(2-methoxyethyl)-4-nitro-1*H*-pyrazole-3-carboxamide (20 g, 82.6 mMol) and 5%Pd/C (1 g) in methanol (200 mL) was pressurised at 50psi/25°C in a sealed vessel and stirred for 15 hours. At the end of the reaction the mixture was filtered through arbocel and the filter cake was washed with methanol. The methanolic solution was distilled at atmospheric pressure and replaced with ethyl acetate to a final volume of 100 mL. The cooled mixture was granulated at ambient temperature for 2 h filtered and washed with ethyl acetate (20 mL) to afford 4-amino-5-ethyl-1-(2-methoxyethyl)-1*H*-pyrazole-3-carboxamide as a white crystalline solid, 15 g, 88%. m.p. = 131°C. Found: C, 50.75; H, 7.62; N, 26.38. C₉H₁₆N₄O₂ requires C, 50.94; H, 7.55; N, 26.42%.
δ(CDCl₃): 1.20 (3H, t), 2.63 (2H, q), 3.32 (3H, s), 3.74 (2H, t), 3.95 (2H, s), 4.15 (2H, t), 5.27 (1H, s), 6.59 (1H, s).

LRMS: m/z = 213 (M+1)⁺

### (i) N-[3-Carbamoyl-5-ethyl-1-(2-methoxyethyl)-1H-pyrazol-4-yl}-2-ethoxy-5-(4-ethyl-1-piperazinyl sulfonyl) nicotinamide.

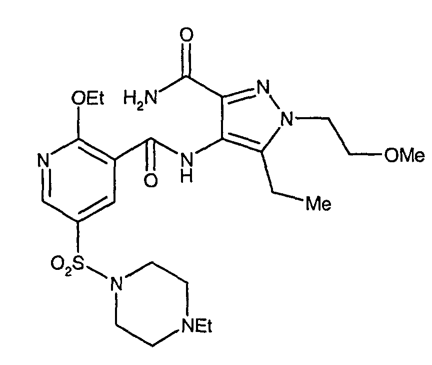

2-ethoxy-5-(4-ethyl-1-piperazinylsulfonyl)nicotinic acid (2.31 kg, 6.73 Mol) was suspended in ethyl acetate (16.2 L) and 1,1-carbonyldimidazole (1.09 kg, 6.73 Mol) was added at room temperature. The reaction mixture was heated at 45°C for 40 minutes and then the reaction was stirred for a further 40 minutes at reflux. After cooling to ambient temperature 4-amino-5-ethyl-1-(2-methoxyethyl)-1H-pyrazole-3-carboxamide (1.5 kg, 7.06 Mol) was added to the cooled mixture, and the reaction stirred for a further 15 hours under reflux. The mixture was cooled filtered and the filter cake was washed with 90% water / 10% ethyl acetate, (2 mL /g) to afford N-[3-carbamoyl-5-ethyl-1-(2-methoxyethyl)-1*H*-pyrazol-4-yl}-2-ethoxy-5-(4-ethyl-1-piperazinyl sulfonyl) nicotinamide as an off white crystalline solid, 3.16 kg, 88%. m.p. = 156°C. Found: C, 51.33; H, 6.56; N, 18.36. C₂₃H₃₅N₇O₆S requires C, 51.40; H, 6.53; N, 18.25%.
δ(CDCl₃): 1.04 (3H, t), 1.22 (3H, t), 1.60 (3H, t), 2.44 (2H, q), 2.54 (4H, m), 2.96 (2H, q), 3.12 (4H, m), 3.36 (3H, s), 3.81 (2H, t), 4.27 (2H, t), 4.80(2H, q), 5.35(1 H, s), 6.68 (1H, s), 8.66 (1H, d), 8.86 (1H, d), 10.51 (1H, s).
LRMS: m/z = 539 (M+1)⁺

### (i) 1-(6-Ethoxy-5-[3-ethyl]-6,7-dihvdro-2-(2-methoxyethyl)-7-oxo-2H-pyrazole[4,3-d]pyrimidin-5-yl]-3-pyridylsulfonyl)-4-ethylpiperazine•ethyl acetate solvate.

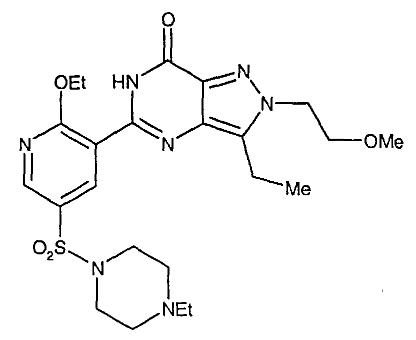

A mixture of *N*-[3-carbamoyl-5-ethyl-1-(2-methoxyethyl)-1*H*-pyrazol-4-yl}-2-ethoxy-5-(4-ethyl-1-piperazinyl sulfonyl) nicotinamide (1.18 kg, 2.2 Mol), potassium *tert*-butoxide (500 g, 4.4 moles) and ethyl acetate (193 g) in ethanol (11.8 L) was heated at 120°C for 20 hours. The reaction mixture was then concentrated under reduced pressure, in total approx. 10 L of solvent were distilled. To the residue water (2.9 L) was added and the mixture stirred at room temperature while aqueous HCI was added until pH 7.5 was obtained. Ethyl acetate (7.5 L) was added and the two phase mixture was warmed to 55°C. The organic phase was separated and the aqueous phase was extracted with further ethyl acetate (3.0 L). The combined organic phases were distilled at atmospheric pressure to a final volume of 4L. The precipitated solids were granulated at 5°C for 1 h, filtered and washed with ethyl acetate (1.2 L) and dried under vacuum. This afforded 1-(6-Ethoxy-5-[3-ethyl]-6,7-dihydro-2-(2-methoxyethyl)-7-oxo-2H-pyrazole[4,3-*d*]pyrimidin-5-yl]-3-pyridylsulfonyl)-4-ethylpiperazine as a light yellow crystalline solid, 877 g, 78%. m.p. = 157°C. Found: C, 52.65; H, 6.46; N, 17.76. C₂₃H₃₃N₇O₅S. 0.2 C₂H₅CO₂CH₃ requires C, 53.21; H, 6.49; N, 18.25%.
δ(CDCl₃): 1.07 (3H, t), 1.42 (3H, t), 1.61 (3H, t), 2.44 (2H, q), 2.57 (4H, m), 3.08 (2H, q), 3.15 (4H, m), 3.32 (3H, s), 3.92 (2H, q), 4.48 (2H, q), 4.77 (2H, q), 8.65 (1H, d), 9.06 (1H, d). The spectrum also has signals that correspond to a solvate with ethyl acetate.
LRMS: m/z = 520 (M+1)⁺

### Example 2

### 5-(5-acetyl-2-butoxy-3-pyridinyl)-3-ethyl-2-(1-ethyl-3-azetidinyl)-2,6-dihydro-7H-pyrazolo[4,3-d]pyrimidin-7-one.

The title compound from Preparation 2(a) (120 mg, 0.28 mmol) and cesium carbonate (274 mg, 0.84 mmol) were dissolved in n-butanol (4 ml), and heated at 90°C under nitrogen with molecular sieves for 96h. The mixture was then partitioned between water (10 ml) and dichloromethane (10 ml). The organic layer was separated, and the aqueous layer extracted further with dichloromethane (3 x 15 ml). The combined organic layers were dried (MgSO₄), and concentrated *in vacuo*. The crude product was purified by flash column chromatography (95:5:0.5-90:10:1 ethyl acetate:methanol:0.88 NH₃ as eluents), to yield the title compound as a colourless glass (77 mg, 0.18 mmol).
**m.p.** 91.6-93.7°C
**1H NMR** (400MHz, CDCl₃): δ = 1.00-1.05 (m, 6H), 1.38 (t, 3H), 1.50-1.62 (m, 2H), 1.90-2.00 (m, 2H), 2.63 (s, 3H), 2.63-2.70 (m, 2H), 3.02 (q, 2H), 3.75 (t, 2H), 3.90 (t, 2H), 4.68 (t, 2H), 5.10-5.20 (m, 1H), 8.84 (s, 1H), 9.23 (s, 1H), 10.63 (br s, 1H).
**LRMS** (TSP - positive ion) 439 (MH⁺)
**Anal.** Found C, 60.73; H, 7.06; N, 18.03 Calcd for C₂₃H₃₀O₃N₆.0.2MeOH.0.1 DIPE: C, 60.88; H, 7.26; N, 17.90

### Preparation of starting materials for Example 2

### 2(a) 5-(5-Acetyl-2-propoxy-3-pyridinyl)-3-ethyl-2-(1-ethyl-3-azetidinyl)-2,6-dihydro-7H-pyrazolo[4.3-d]pyrimidin-7-one

Sodium cyanoborohydride (92 mg, 1.47 mmol) was added to a stirring solution of title compound from Preparation 2(b) (500 mg, 0.98 mmol), acetaldehyde (64µl, 1.18 mmol) and sodium acetate (161 mg, 1.96 mmol) in methanol (10 ml) under nitrogen at room temperature. After 1h the mixture was poured into NaHCO₃ (sat. aq., 20 ml), and extracted with dichloromethane (3 x 15 ml). The combined organic layers were dried (MgSO₄) and concentrated *in vacuo.* The crude product was purified by flash column chromatography (95:5:0.5-80:20:1 ethyl acetate:methanol:0.88 NH₃ as eluent) to yield the title compound as a white solid (140 mg, 0.33 mmol).
**1H NMR** (400MHz, CDCl₃): δ = 0.97 (t, 3H), 1.03 (t, 3H), 1.30 (t, 3H), 2.82-2.97 (m, 2H), 2.58-2.65 (m, 5H), 2.98 (q, 2H), 3.68 (t, 2H), 3.85 (dd, 2H), 4.58 (dd, 2H), 5.05-5.17 (m, 1H), 8.79 (s, 1H), 9.18 (s, 1H), 10.62 (br s, 1H).
**LRMS** (TSP - positive ion) 426 (MH⁺)

### 2(b) 5-(5-Acetyl-2-propoxy-3-pyridinyl)-2-(3-azetidinyl)-3-ethyl-2,6-dihydro-7H-pyrazolo[4,3-d]pyrimidin-7-one

The title compound of Preparation 2(c) (1.44 g, 3.0 mmol) in acetone (50 ml) and sulphuric acid (1N, 3 ml) was treated with mercuric sulphate (268 mg, 9.0 mmol) and heated to reflux for 6h. The reaction mixture was concentrated to ∼20 ml *in vacuo*, poured into sodium bicarbonate (sat. aq., 20ml) and extracted into methylene chloride (6 x 20 ml). Combined organics were washed with brine (20 ml), dried over MgSO₄, and concentrated to a brown oil which was taken up in 40% trifluoroacetic acid in methylene chloride (50ml) and water (1 ml) and stirred for 1h at room temperature. After evaporation *in vacuo,* the residue was purified by column chromatography (eluting with 95:5:1 methylene chloride:methanol:0.88 ammonia) to afford the title compound as a white hydroscopic foam (1.65 g).
**m.p.** 128.5-130.0°C
**1H NMR** (400MHz, MeOD): δ = 1.00 (t, 3H), 1.30 (t, 3H), 1.79-1.90 (m, 2H), 2.60 (s, 3H), 3.00-3.10 (q, 2H), 4.50 (t, 2H), 4.60-4.70 (m, 4H), 5.65-5.78 (m, 1H), 8.65 (s, 1H), 8.90 (s, 1H)
**LRMS** (TSP - positive ion) 397 (MH⁺)

### 2(c) tert-Butyl 3-[3-ethyl-5-(5-ethynyl-2-propoxy-3-pyridinyl)-7-oxo-6,7-dihydro-2H-pyrazolo[4,3-d]pyrimidin-2-yl]-1-azetidinecarboxylate

Prepared from the title compound of Preparation 2(d) by the method of Preparation 2(c)(i).
**1H NMR** (400MHz, CDCl₃): δ = 1.05 (t, 3H), 1.30 (t, 3H), 1.43 (s, 9H), 1.88-2.00 (m, 2H), 3.00 (q, 2H), 3.19 (s, 1H), 4.35 (app t, 2H), 4.52 (app t, 2H), 4.60-4.80 (br s, 2H), 5.22 (t, 1H), 8.39 (s, 1H), 8.80 (s, 1H), 10.75 (br s, 1H)
**LRMS** (TSP - positive ion) 496 (MNH₄⁺).

### 2(c)(i) 5-(2-Butoxy-5-ethynyl-3-pyridinyl)-3-ethyl-2-(2-methoxyethyl)-2,6-dihydro-7H-pyrazolo[4,3-d]pyrimidin-7-one

Potassium fluoride (22 mg, 0.38 mmol) was added to a stirred solution of the title compound of Preparation 2(d)(i) (90 mg, 0.19 mmol) in aqueous *N,N*-dimethylformamide (2 mL *N*,*N*-dimethylformamide /0.2 mL water) at 0°C. After 10 min the reaction was allowed to warm to room temperature and stirred for 2 h. The reaction mixture was diluted with ethyl acetate and washed with water, 1 N hydrochloric acid (3 times) and brine. The organic layer was dried (MgSO₄) and concentrated to give the title compound as a white solid (75 mg).
^{**1**}**H NMR** (400 MHz, CDCl₃): δ = 1.00 (t, 3H), 1.40 (t, 3H), 1.50 (m, 2H), 1.90 (m, 2H), 3.05 (q, 2H), 3.20 (s, 1H), 3.30 (s, 3H), 3.85 (t, 2H), 4.40 (t, 2H), 4.60 (t, 2H), 8.40 (s, 1H), 8.80 (s, 1H), 10.70 (s, 1H).
**LRMS** (TSP): 396.3 (MH⁺).

### 2(d) tert-Butyl 3-(3-ethyl-7-oxo-5-{2-propoxv-5-[(trimethylsilyl)ethynyl]-3-pyridinyl}-6,7-dihydro-2H-pyrazolo[4,3-d]pyrimidin-2-yl)-1-azetidinecarboxylate

Prepared from the title compound of Preparation 2(e) by the method of Preparation 2(d)(i).
**1H NMR** (400MHz, MeOD): δ = 0.25 (s, 9H), 1.05 (t, 3H), 1.31 (t, 3H), 1.44 (s, 9H), 1.87-1.96 (m, 2H), 3.00 (q, 2H), 4.33 (t, 2H), 4.52 (t, 2H), 4.54-4.80 (m, 2H), 5.18-5.25 (m, 1H), 8.32 (d, 1H), 8.74 (d, 1H)
**LRMS** (TSP - positive ion) 569 (MNH₄⁺), 552.0 (MH⁺)
**Anal.** Found C, 60.82; H, 6.90; N, 15.15 Calcd for C₂₈H₃₈O₄N₆Si: C, 61.07; H, 6.95; N, 15.26.

### 2(d)(i) 5-(2-Butoxy-5-trimethylsilylethynyl-3-pyridinyl)-3-ethyl-2-(2-methoxyethyl)-2,6-dihydro-7H-pyrazolo[4,3-d]pyrimidin-7-one

The title compound from Example 1 of PCT application IB00/1430 (127 mg, 0.25 mmol) was suspended in triethylamine (2 mL) and trimethylsilylacetylene (38 mg, 0.39 mmol) and acetonitrile (2 mL). Pd(PPh₃)₂Cl₂ (5 mg, 0.006 mmol) and cuprous iodide (1.2 mg, 0.006 mmol) were added and the reaction mixture stirred. After 1 h a further portion of trimethylsilylacetylene (19 mg, 0.19 mmol) was added and stirring continued for 2 h. The solvent was evaporated and the residue partitioned between ethyl acetate and water. The organics were washed with brine, dried (MgSO₄) and concentrated to give a brown foam. Purification by flash column chromatography (gradient elution from 100% dichloromethane to 99% dichloromethane/methanol) gave the title compound as a light brown solid (108 mg).
^{**1**}**H NMR** (300 MHz, CDCl₃): δ = 0.25 (s, 9H), 1.00 (t, 3H), 1.40 (t, 3H), 1.50 (m, 2H), 1.90 (m, 2H), 3.10 (q, 2H), 3.30 (s, 3H), 3.90 (t, 2H), 4.40 (t, 2H), 4.60 (t, 2H), 8.40 (s, 1H), 8.80 (s, 1H), 10.70 (s, 1H).
**LRMS** (TSP): 468.3 (MH⁺).

### 2(e) tert-Butyl 3-[3-ethyl-5-(5-iodo-2-propoxy-3-pyridinyl)-7-oxo-6,7-dihydro-2H-pyrazolo[4,3-d]pyrimidin-2-yl]-1-azetidinecarboxylate

The title compound was prepared from the product of Preparation 2(f) using the method of Preparation 2(e)(i).
**1H NMR** (400MHz, CDCl₃): δ = 1.05 (t, 3H), 1.30 (t, 3H), 1.43 (s, 9H), 1.87-1.96 (m, 2H), 3.00 (q, 2H), 4.34 (t, 2H), 4.49 (t, 2H), 4.60 (br s, 2H), 5.20 (t, 1H), 8.41 (d, 1H), 8.94 (s, 1H), 10.75 (br s, 1H)
**LRMS** (TSP - positive ion) 598.1 (MNH₄⁺)
**Anal.** Found C, 47.54; H, 5.02; N, 14.09 Calcd for C₂₃H₂₉O₄N₆I: C, 47.60; H, 5.04; N, 14.48.

### 2(e)(i) 3-Ethyl-5-(5-iodo-2-propoxy-3-pyridinyl)-1-[2-(4-morpholinyl)ethyl]-1,6-dihydro-7H-pyrazolo[4,3-d]pyrimidin-7-one

The title compound of Preparation 48 of PCT application IB00/1430 (15.78 g, 28.4 mmol) was dissolved in n-propanol (200 ml), ethyl acetate (6 ml) and potassium t-butoxide (3.2 g, 28.4 mmol) were added and the resultant mixture heated to reflux for 6h. Additional potassium t-butoxide (1.6 g, 14.2 mmol) was added and the mixture heated for a further 2h, after which the solvent was removed *in vacuo*. The residue was partitioned between water (50 ml) and methylene chloride (100 ml) and the organic phase separated. The aqueous phase was extracted with dichloromethane (2 x 100 ml) and the combined organics dried over MgSO₄ and reduced to a yellow solid (∼17 g). Purification by column chromatography (elution with ethyl acetate) gave the title compound (13.3 g, 24.1 mmol) together with recovered starting material (2.31 g, 4.2 mmol).
**m.p.** 175-177°C.
**1H NMR** (300 MHz, CDCl₃): δ = 1.1 (t, 3H), 1.4 (t, 3H), 1.9-2.05 (m, 2H), 2.45-2.55 (m, 4H), 2. 85 (t, 2H), 3.0 (q, 2H), 3.6-3.65 (m, 4H), 4.5 (t, 2H), 4.7 (t, 2H), 8.4 (s, 1H), 9.0 (s, 1H), 10.95 (br s, 1H).
**LRMS** (TSP) 540 (MH⁺).
**Analysis:** found C, 46.79; H, 5.01; N, 15.44. Calcd for C₂₁H₂₇N₆O₃I : C, 46.85; H, 5.05; N, 15.61%

### 2(f) tert-Butyl 3-(3-(aminocarbonyl)-5-ethyl-4-{[5-iodo-2-propoxy-3-pyridinyl)carbonyl]amino}-1H-pyrazol-1-yl)-1-azetidinecarboxylate

The title compound was prepared by the method of Preparation 2(f)(i) using the products from Preparations 2(g) and 2(i).
**1H NMR** (400MHz, DMSO): δ = 0.95 (t, 3H), 1.05 (t, 3H), 1.40 (s, 9H), 1.78-1.88 (m, 2H), 2.68 (q, 2H), 4.22-4.35 (m, 4H), 4.40 (t, 2H), 5.33 (t, 1H), 7.35 (bs, 1H), 7.52 (bs, 1H), 8.40 (s, 1H), 8.55 (s, 1H), 10.10 (s, 1H)
**LRMS** (TSP - positive ion) 373.2 (MH⁺- BOC and I)
**Anal.** Found C, 45.11; H, 5.07; N, 13.56 Calcd for C₂₃H₃₁O₅N₆I. 0.2 DCM: C, 45.28; H, 5.14; N, 13.66.

### 2(f)(i) N-{3-(Aminocarbonyl)-1-[2-dimethylamino)ethyl]-5-ethyl-1H-pyrazol-4-yl}-2-butoxy-5-iodonicotinamide

Cesium carbonate (1.17 g, 3.59 mmol) was added to a stirred solution of the title compound from Preparation 16 of PCT application IB00/1430 (800 mg, 1.79 mmol) and *N,N*-dimethylaminoethyl chloride hydrochloride (309 mg, 2.15 mmol) in *N*,*N*-dimethylformamide (10 mL) under a nitrogen atmosphere. The mixture was heated at 80°C for 24 h. The mixture was cooled and extracted from water with ethyl acetate. The organics were dried (MgSO₄) and concentrated to give a brown oil. Purification by flash column chromatography (gradient elution from 100% dichloromethane to 90% dichloromethane/MeOH) gave the product as a pale brown oil (522 mg).
^{**1**}**H NMR** (400 MHz, CDCl₃): δ = 0.95 (t, 3H), 1.20 (t, 3H), 1.40 (m, 2H), 1.90 (m, 2H), 2.35 (s, 6H), 2.80 (t, 2H), 2.85 (q, 2H), 4.20 (t, 2H), 4.60 (t, 2H), 5.30 (br s, 1H), 6.60 (br s, 1H), 8.40 (s, 1H), 8.75 (s, 1H), 10.35 (s, 1H).
**LRMS** (TSP): 529.5 (MH⁺).

### 2(g) N-[3-(Aminocarbonyl)-5-ethyl-1H-pyrazol-4-yl]-5-iodo-2-propoxynicotinamide

The title compound was prepared from 2-propoxy-5-iodonicotinic acid (see Preparation 2(h) and 4-amino-3-ethyl-1*H*-pyrazole-5-carboxamide (prepared as described in WO 98/49166) according to the method described in Preparation 2(g)(i).
^{**1**}**H NMR** (300 MHz, d₄-MeOH): δ = 1.0 (t, 3H), 1.25 (t, 3H), 1.85-2.0 (m, 2H), 2.8 (q, 2H), 4.5 (t, 2H), 8.5 (s, 1H), 8.6 (s, 1H).
**LRMS** (TSP) 444 (MH⁺).

### 2(g)(i) N-[3-(Aminocarbonyl)-5-ethyl-1-(2-methoxyethyl)-1H-pyrazol-4-yl]-2

### butoxy-5-iodonicotinamide

Oxalyl chloride (2 g, 15.9 mmol) was added to a stirred solution of the title compound from Preparation 4 of PCT application IB00/1430 (1.28 g, 3.98 mmol) in dichloromethane (20 mL) and 3 drops *N*,*N*-dimethylformamide added. After 2.5 h the solvent was evaporated and the residue azeotroped 3 times with dichloromethane. The residue was resuspended in dichloromethane (4 mL) and added to a stirred mixture of the title compound of Preparation 11 from PCT application IB00/1430 (0.76 g, 3.58 mmol) and triethylamine (0.8 g, 7.97 mmol) in dichloromethane (10 mL). After 1 h the solvent was evaporated and the residue partitioned between ethyl acetate and water. The organic phase was separated and washed with 2N HCl (twice), sodium bicarbonate solution (twice) and brine before being dried (MgSO₄) and concentrated. The product was triturated with ether and filtered to give 820 mg of pure product as a white solid. The mother liquor was concentrated and purified by flash column chromatography (elution with 80% ethyl acetate : hexane), to give a further 605 mg of product.
^{**1**}**H NMR** (400 MHz, CDCl₃): δ = 0.95 (t, 3H), 1.20 (t, 3H), 1.45 (m, 2H), 1.90 (m, 2H), 2.85 (q, 2H), 3.35 (s, 3H), 3.80 (t, 2H), 4.25 (t, 2H), 4.60 (t, 2H), 5.20 (br s, 1H), 6.60 (br s, 1H), 8.40 (s, 1H), 8.80 (s, 1H), 10.30 (s, 1H).
**LRMS** (TSP): 516.2 (MH⁺).

### 2(h) 2-Propoxy-5-iodonicotinic acid

The title compound was prepared from 2-propoxy nicotinic acid (prepared as described in WO 99/54333, the compound 2-n-propoxypyridine-3-carboxylic acid, Preparation 46 prepared by the process of Preparation 1) using the method of Preparation 2(h)(i).
^{**1**}**H NMR** (300 MHz, CDCl₃): δ = 1.05 (t, 3H), 1.85-2.0 (m, 2H), 4.5 (t, 2H), 8.5 (s, 1H), 8.6 (s, 1H).
**Analysis:** found C, 35.16; H, 3.19; N, 4.46. Calcd for C₉H₁₀INO₃: C, 35.19; H, 3.28; N, 4.56%

### 2(h)(i) 2-isoButoxy-5-iodo nicotinic acid

*N*-Iodosuccinamide (18.22 g, 0.08 mol), trifluoroacetic acid (100 mL) and trifluoroacetic anhydride (25 mL) were added to 2-isobutoxynicotinic acid (10.55 g, 0.054 mol). The mixture was refluxed for 2.5 h, cooled and the solvents evaporated. The residue was extracted from water with ethyl acetate and the organics washed with water (twice) and brine (twice), dried (MgSO₄) and concentrated. The red residue was redissolved in ethyl acetate washed with sodium thiosulfate solution (twice), water (twice), brine (twice), redried (MgSO₄) and concentrated to give the desired product as a yellow solid.
^{**1**}**H NMR** (300 MHz, CDCl₃): δ = 1.05 (d, 6H), 2.20 (m, 1H), 4.40 (d, 2H), 8.50 (s, 1H), 8.70 (s, 1H),
**LRMS** (TSP): 322.3 (MH⁺).

### 2(i) tert-Butyl 3-iodo-1-azetidinecarboxylate

A mixture of *tert*-butyl 3-[(methylsulfonyl)oxy]-1-azetidinecarboxylate (prepared as described in *Synlett* **1998,** 379; 5.0 g, 19.9 mmol), and potassium iodide (16.5 g, 99.4 mmol) in *N*,*N*-dimethylformamide (25 mL), was heated at 100°C for 42 h. The cooled mixture was partitioned between water and ethyl acetate, and the layers separated. The organic phase was dried over MgSO₄, concentrated under reduced pressure and the residue azeotroped with xylene. The crude product was purified by flash column chromatography (dichloromethane as eluant) to give the title compound, 3.26 g.
^{**1**}**H NMR** (300 MHz, CDCl₃) δ = 1.43 (s, 9H), 4.28 (m, 2H), 4.46 (m, 1H), 4.62 (m, 2H).
**LRMS** (TSP) 284 (MH)⁺

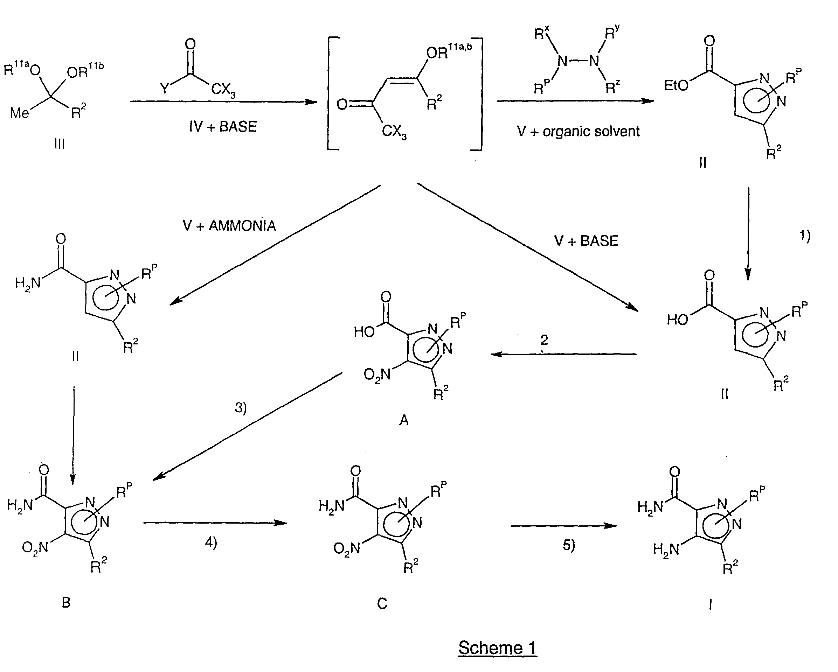

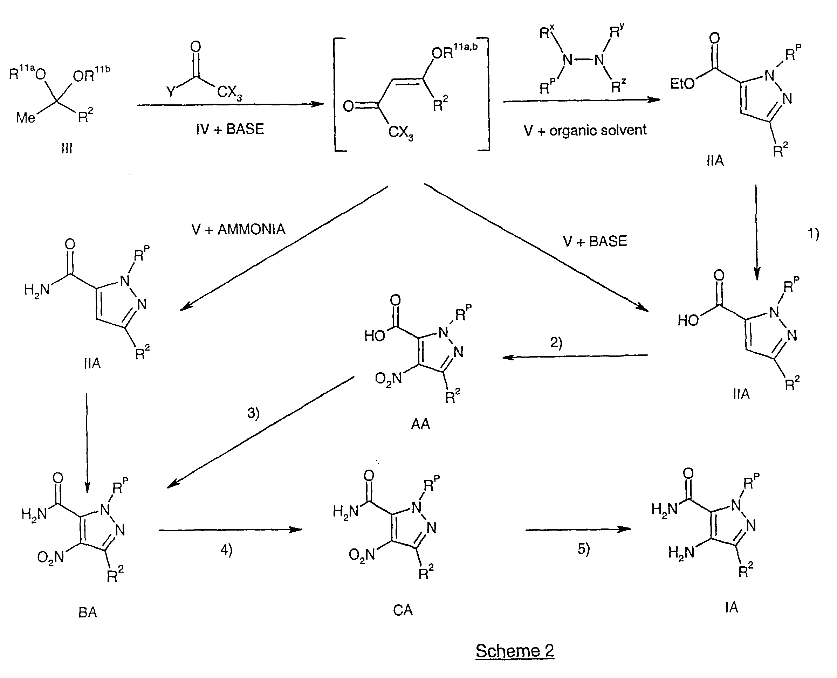

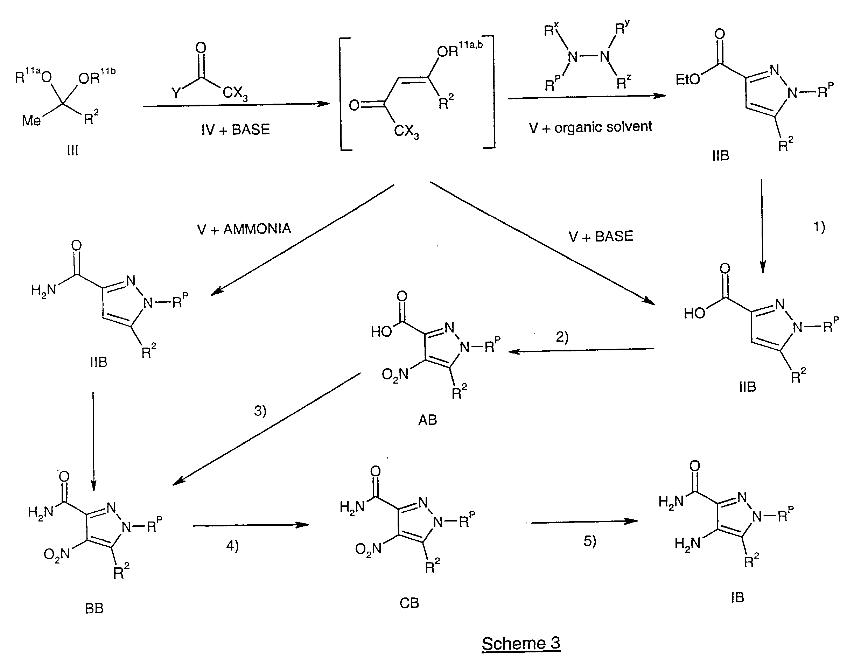

## Claims

1. A process for the production of pyrazole compounds of general formula (II): 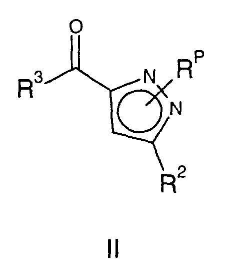 wherein R^{P} represents H or R¹;
R¹ represents: H, C₁-C₆ alkyl; C₁-C₆ alkoxy; C₃-C₆ cycloalkyl; C₁-C₆ alkyl(C₁-C₆ alkoxy), Het, C₁-C₆ alkylHet, aryl or C₁-C₆ alkylaryl, which latter eight groups are all optionally substituted (and/or, in the case of C₁-C₆ alkyl, optionally terminated) by one or more substituents selected from halo, cyano, nitro, C₁-C₆ alkyl, C(O)NR⁴R⁵, C(O)R⁶, C(O)OR⁷, OR⁸, NR^{9a}R^{9b} and SO₂NR^{10a}R^{10b};
R² represents C₁-C₆ alkyl; C₁-C₆ alkoxy; C₃-C₆ cycloalkyl; C₁-C₆ alkyl(C₁-C₆ alkoxy), Het, C₁-C₆ alkylHet, aryl or C₁-C₆ alkylaryl, which latter eight groups are all optionally substituted (and/or, in the case of C₁-C₆ alkyl, optionally terminated) by one or more substituents selected from halo, cyano, nitro, C₁-C₆ alkyl, C(O)NR⁴R⁵, C(O)R⁶, C(O)OR⁷, OR⁸, NR^{9a}R^{9b} and SO₂NR^{10a}R^{10b};
R³ represents: OH, C₁-C₆ alkoxy or NR⁴R⁵;
R⁴, R⁵, R⁶, R⁷, R⁸, R^{10a} and R^{10b} independently represent H or C₁-C₆ alkyl;
R^{9a} and R^{9b} either independently represent, H or C₁-C₆ alkyl or, together with the nitrogen atom to which they are attached, represent azetidinyl, pyrollidinyl or piperidinyl,
which process comprises the reaction of a compound of general formula (III), 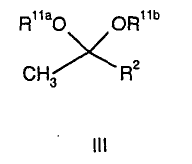 wherein R^{11a} and R^{11b} independently represent C₁-C₆ alkyl, R² is as defined herein before,
with an acylating agent of the formula (IV) in the presence of a base, 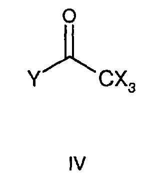 wherein X represents halogen independently selected from Cl, F or Br and wherein Y represents halogen or OR¹² wherein R¹² is C₁-C₆ alkyl, C(O)CX₃, Het, or C₀-C₆ alkyl(Het) wherein Het is pyridine or imidazole, followed by *in situ* reaction with a hydrazine compound of general formula (V) 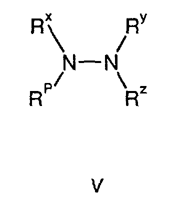 wherein R^{p} represents H or R¹ wherein R¹ is as defined hereinbefore, and R^{x}, R^{y} and R^{z} are independently selected from H or electron donating groups (EDG) or electron withdrawing groups (EWG) wherein said electron withdrawing or donating groups are labile under the conditions of the reaction,
wherein said process is optionally carried out in the presence of an activating agent.

2. A process for the preparation of compounds of formula (I): 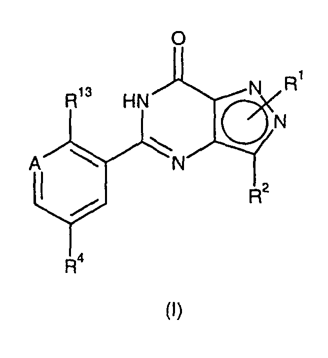 or a pharmaceutically or veterinarily acceptable salt thereof, or a pharmaceutically or veterinarily acceptable solvate of either entity,
wherein:
A is CH or N;
R¹ represents: H, C₁-C₆ alkyl; C₁-C₆ alkoxy; C₃-C₆ cycloalkyl; C₁-C₆ alkyl(C₁-C₆ alkoxy), Het, C₁-C₆ alkylHet, aryl or C₁-C₆ alkylaryl, which latter eight groups are all optionally substituted (and/or, in the case of C₁-C₆ alkyl, optionally terminated) by one or more substituents selected from halo, cyano, nitro, C₁-C₆ alkyl, C(O)NR⁴R⁵, C(O)R⁶, C(O)OR⁷, OR⁸, NR^{9a}R^{9b} and SO₂NR^{10a}R^{10b};
R² represents C₁-C₆ alkyl; C₁-C₆ alkoxy; C₃-C₆ cycloalkyl; C₁-C₆ alkyl(C₁-C₆ alkoxy), Het, C₁-C₆ alkylHet, aryl or C₁-C₆ alkylaryl, which latter eight groups are all optionally substituted (and/or, in the case of C₁-C₆ alkyl, optionally terminated) by one or more substituents selected from halo, cyano, nitro, C₁-C₆ alkyl, C(O)NR⁴R⁵, C(O)R⁶, C(O)OR⁷, OR⁸, NR^{9a}R^{9b} and SO₂NR^{10a}R^{10b};
R^{t} represents: NR^{p}R^{q};
R^{p}, R^{q}, R⁶, R⁷, R⁸, R^{10a} and R^{10b} independently represent H or C₁-C₆ alkyl;
R^{9a} and R^{9b} either independently represent, H or C₁-C₆ alkyl or, together with the nitrogen atom to which they are attached, represent azetidinyl, pyrollidinyl or piperidinyl;
R⁴ is C₁-C₄ alkyl optionally substituted with OH, NR⁵R⁶, CN, CONR⁵R⁶ or CO₂R⁷; or R⁴ is CO₂R⁷; or R⁴ is a pyrrolidinylsulphonyl, piperidinosulphonyl, morpholinosulphonyl, or piperazin-1-ylsulphonyl group having a substituent, R¹⁰ at the 4-position of the piperazinyl group wherein said piperazinyl group is optionally substituted with one or two C₁ to C₄ alkyl, C₁ to C₃ alkoxy, NR⁷R⁸ or CON R⁷R⁸ groups and is optionally in the form of its 4-N-oxide;
R¹⁰ represents H or C₁-C₆ alkyl;
wherein said compounds may be prepared from compounds of general formula (VIII): 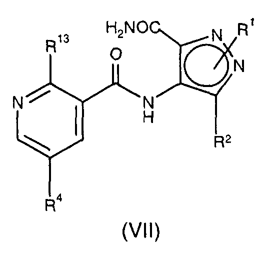 wherein R¹, R², R⁴ and R¹³ are as defined hereinbefore wherein said compound of general formula (VIII) is prepared from the reaction of a compound of general formula (VII): 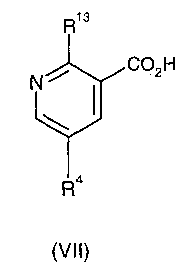 wherein R⁴ and R¹³ are as defined herein before via coupling with a compound of general formula (VI): 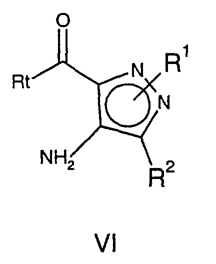 wherein R¹ and R² are as defined hereinbefore and wherein R^{t} is NR^{p}R^{q} wherein R^{p} and R^{q} independently represent H or C₁-C₆ alkyl and wherein said compound of general formula (VI) is prepared by nitration and hydrogenation of a compound of general formula (II): 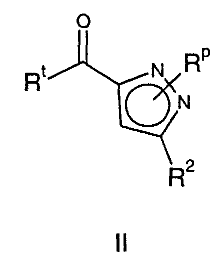 wherein R^{t} and R² are as defined hereinbefore and wherein R^{P} represents R¹ as defined hereinbefore
**characterised in that** said compound of general formula (II) is prepared from the reaction of a compound of general formula (III), 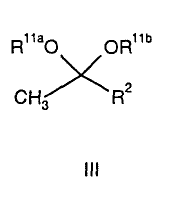 wherein R^{11a} and R^{11b} independently represent C₁-C₆ alkyl, R² is as defined herein before,
with an acylating agent of the formula (IV) in the presence of a base, 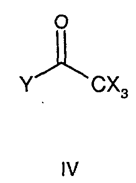 wherein X represents halogen independently selected from Cl, F or Br, and Y represents halogen or OR¹² wherein R¹² is: C₁-C₆ alkyl; C(O)CX₃; Het; or C₀-C₆ alkyl(Het) wherein Het is pyridine or imidazole,
followed by *in situ* reaction with a hydrazine compound of general formula (V) 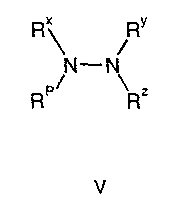 wherein R^{P} represents H or R¹ wherein R¹ is as defined hereinbefore, and R^{x}, R^{y} and R^{z} are independently selected from H or electron donating groups (EDG) or electron withdrawing groups (EWG) wherein said electron withdrawing or donating groups are labile under the conditions of the reaction,
wherein said process for the preparation of compound (II) is optionally carried out in the presence of an activating agent.

3. A process according to claim 2 wherein the compound of formula (I) is sildenafil, 5-[2-Ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one, or 5-(5-acetyl-2-butoxy-3-pyridinyl)-3-ethyl-2-(1-ethyl-2-(1-ethyl-3-azetidinyl)-2,6-dihydro-7*H*-pyrazolo[4,3-d]pyrimidin-7-one.
